# EUROPEAN PATENT APPLICATION

(11) **EP 4 792 870 A1**
(43) Date of publication of application: **19.08.2026**
(21) Application number: 26158374.4
(22) Date of filing: 13.02.2026
(51) Int. Cl.: A61N 1/05, A61N 1/372, A61N 1/375

(54) **BIOSTIMULATOR HAVING A TAPERING FIXATION ELEMENT**

(30) Priority: 18.02.2025 US 202563759923 P; 11.02.2026 US 202619537312
(71) Applicant: Pacesetter, Inc., Sylmar, CA 91342 (US)
(72) Inventor: VICTORINE, Keith, Sylmar, CA 91342 (US)
(74) Representative: Barker Brettell Sweden AB

(57) **Abstract**

A biostimulator (100) includes a housing (302) having a longitudinal axis (304) and containing an electronics compartment (306). The biostimulator (100) includes a fixation element mount (307) mounted on the housing (302). The biostimulator (100) includes a fixation element (106) mounted on the fixation element mount (307). The fixation element (106) includes a helical wire (320) extending helically about the longitudinal axis (304). The helical wire (304) tapers inward from a proximal turn (402) to a distal tip (322).

## Description

### TECHNICAL FIELD

The present disclosure relates to biostimulators. More specifically, the present disclosure relates to leadless biostimulators useful for atrial pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right atrium and, for dual-chamber pacing, in a right ventricle, by an anchor.

### SUMMARY

Atrial pacing can be performed using a dual-helix fixation design that combines a helical anchor surrounding a helical pacing electrode. When screwing the dual-helix structure into a thin atrial wall, however, the helical anchor can traumatize tissue surrounding the tissue engaged by the helical pacing electrode. The surrounding tissue can therefore act like a buffer that blocks conduction pathways and increases capture thresholds. The buffer may inhibit propagation of pacing impulses to a target tissue zone. Accordingly, a fixation design that combines fixation and pacing in a single fixation element to engage and pace a thin atrial wall would be beneficial.

A biostimulator is described. In an embodiment, the biostimulator includes a housing having a longitudinal axis and containing an electronics compartment. The biostimulator includes a fixation element mount mounted on the housing. The biostimulator includes a fixation element mounted on the fixation element mount. The fixation element includes a helical wire extending helically about the longitudinal axis. The helical wire tapers inward from a proximal turn to a distal tip.

A biostimulator system is described. In an embodiment, the biostimulator system includes a biostimulator transport system. The biostimulator system includes a biostimulator. The biostimulator includes a housing having a longitudinal axis and containing an electronics compartment. The biostimulator includes a fixation element mount mounted on the housing. The biostimulator includes a fixation element mounted on the fixation element mount. The fixation element includes a helical wire extending helically about the longitudinal axis. The helical wire tapers inward from a proximal turn to a distal tip.

The above summary does not include an exhaustive list of all aspects of the present invention. It is contemplated that the invention includes all systems and devices that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator transport system of a biostimulator system, in accordance with an embodiment.
FIG. 3 is a side view of a biostimulator having a tapering fixation element, in accordance with an embodiment.
FIG. 4 is a perspective view of a distal portion of a biostimulator having a tapering fixation element, in accordance with an embodiment.
FIG. 5 is a side view of a distal portion of a biostimulator having a tapering fixation element, in accordance with an embodiment.
FIG. 6 is an end view of a biostimulator having a tapering fixation element, in accordance with an embodiment.

### DETAILED DESCRIPTION

Embodiments describe a biostimulator, such as a leadless pacemaker, having a tapered fixation element. As described below, the biostimulator can be used to perform atrial pacing of a heart. The biostimulator may, however, be used in other applications, such as deep brain stimulation. Thus, reference to the biostimulator as being a cardiac pacemaker for atrial pacing is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a tapering fixation element. The tapering fixation element provides a hybrid fixation design that can both affix the biostimulator to atrial tissue and pace the tissue. More particularly, the fixation element can include a pacing electrode. The pacing electrode can have a small surface area at a distal tip of the fixation element, which narrows from a wide base, and can therefore provide a high impedance value and a central pacing location near a longitudinal axis. Accordingly, the high impedance pacing tip can reduce capture thresholds of the atrial tissue, providing a single fixation element that both physically affixes the biostimulator to tissue and effectively paces the target tissue at the implant location.

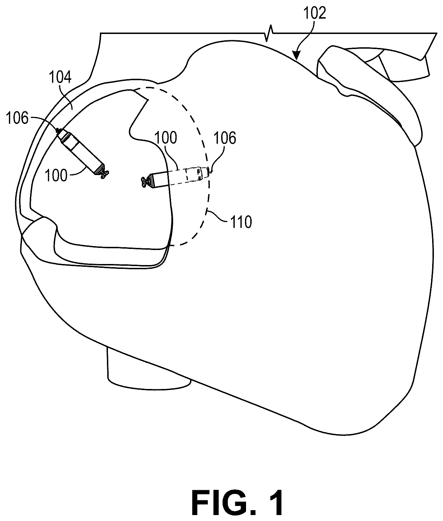

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to an atrial wall 104 of the heart 102. More particularly, the biostimulator 100 can be delivered to the atrial wall 104, which may be a free wall of the atrium. Optionally, one or more biostimulators 100 may be affixed to another atrial wall 104, e.g., a septal wall 110. One or more elements, such as a fixation element 106, can pierce the atrial wall 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, the fixation element 106 is an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 106, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

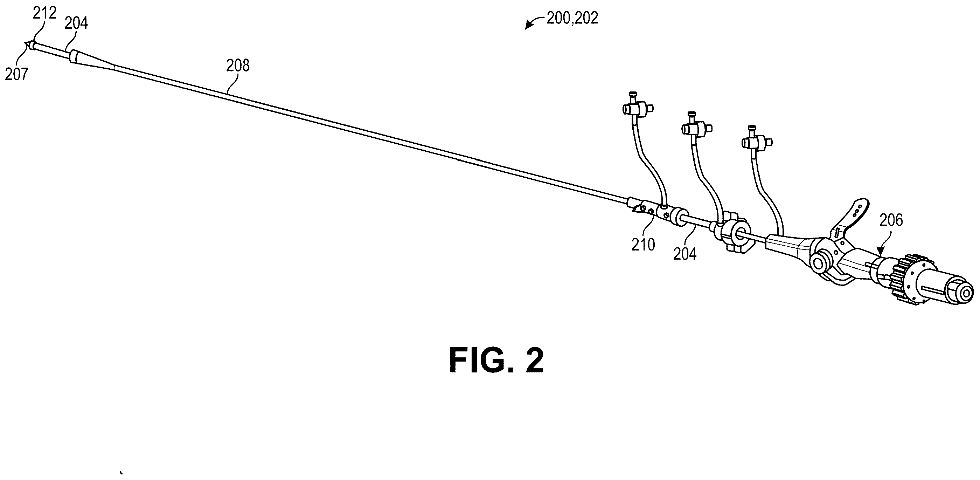

Referring to FIG. 2, a perspective view of a biostimulator transport system of a biostimulator system is shown in accordance with an embodiment. A biostimulator system 200 includes the biostimulator 100 (not shown) mounted on a biostimulator transport system 202. As described above, the biostimulator 100 can be delivered to and retrieved from a patient anatomy using the biostimulator transport system 202. In some implementations, the biostimulator transport system 202 is a delivery system for delivering the leadless pacemaker to the target tissue. In some implementations, the biostimulator transport system 202 is a retrieval system for retrieving the leadless pacemaker from the target tissue. The biostimulator transport system 202 can include a release mechanism to retain the biostimulator 100 described below in an unreleased state and to transition into a released state to release the biostimulator into the target anatomy.

The biostimulator transport system 202 can include an elongated catheter 204 extending distally from a handle 206 to a distal catheter end 207. The elongated catheter 204 can be a deflectable catheter, and an operator can use the handle 206 to steer the distal catheter end 207 in the patient. In an embodiment, the biostimulator transport system 202 includes a guide catheter 208 mounted on the elongated catheter 204. The guide catheter 208 can be slidably disposed on the elongated catheter 204 such that a distal portion of the guide catheter 208 can slide distally over the distal catheter end 207 of the elongated catheter 204 and/or the biostimulator 100, which may be mounted on the distal catheter end (not shown). Similarly, the biostimulator transport system 202 can include an introducer hub assembly 210 mounted on the guide catheter 208. The introducer hub assembly 210 can be slidably disposed on the guide catheter 208 such that a distal portion of the introducer hub assembly 210 can slide distally over the distal catheter end 207 of the elongated catheter 204 and/or the distal portion of the guide catheter 208. More particularly, the introducer hub assembly 210 can be inserted into an access sheath to gain access to the patient vasculature, and after access is established, the distal portion of the guide catheter 208 and/or the distal catheter end 207 of the elongated catheter 204 can be advanced through the access sheath into the patient.

The distal catheter end 207 of the elongated catheter 204 may be selectively connectable to the biostimulator 100. More particularly, the biostimulator 100 can be mounted on the distal catheter end 207 of the elongated catheter 204. The biostimulator 100 can be protected by a protective sheath of the distal portion of the guide catheter 208 during delivery and/or retrieval of the biostimulator 100 from the patient. Accordingly, the biostimulator 100 can be advanced into the patient along with the distal catheter end 207.

The leadless pacemaker system can be used to implant one or more biostimulators 100 within an atrium and/or a ventricle of a heart 102 of the patient. Implantation of each biostimulator 100 may be achieved, in part, by endocardial insertion of the biostimulators 100. For example, the elongated catheter 204 of the leadless pacemaker system can include a torque shaft coupled to a docking cap 212. The docking cap 212 can have a docking cavity to receive an attachment feature of the biostimulator 100. The torque shaft can be torqueable and rotation of the torque shaft can rotate the docking cap 212, which can impart rotation to the attachment feature. Accordingly, torque can be transmitted through the torque shaft to rotate the biostimulator 100 in a first direction, e.g., clockwise. Rotating the biostimulator 100 when a fixation element 106 is in contact with the heart tissue can cause the fixation element to screw into the heart tissue and affix the biostimulator 100 to the heart tissue. Similarly, removal and retrieval of the biostimulators 100 may be accomplished endocardially. For example, the torque shaft of the elongated catheter 204 can be rotated in a second direction, e.g., counterclockwise, to transmit torque through the docking cap 212 to the attachment feature to disengage the biostimulator 100 from the heart tissue.

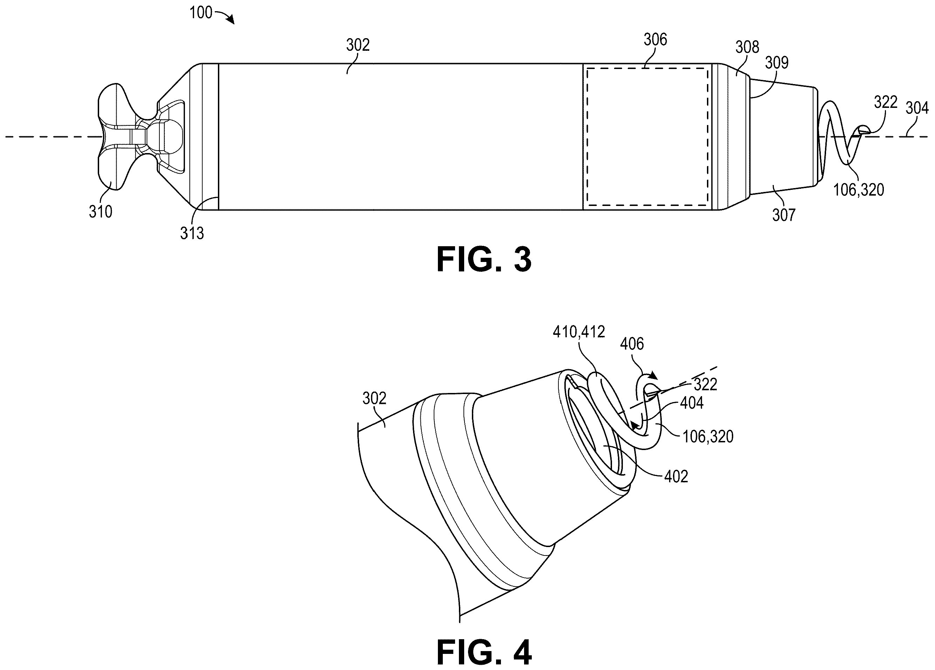

Referring to FIG. 3, a side view of a biostimulator having a tapering fixation element is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., the fixation element 106 that can act as a pacing element and/or a housing 302 that acts as an active electrode. The electrodes can deliver pacing pulses to the atrial wall 104 of the heart 102 to perform atrial pacing, and optionally, can sense electrical activity from the target tissue. The electrodes may also communicate bidirectionally with at least one other device within or outside the body.

In an embodiment, the biostimulator 100 includes the housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold circuitry adapted for different functionality. For example, the electronics compartment 306 can contain circuits for sensing cardiac activity from the electrodes, circuits for receiving information from at least one other device via the electrodes, circuits for generating pacing pulses for delivery to tissue via the electrodes, or other circuitry. The electronics compartment 306 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators can be fixed to an intracardial implant site, e.g., at the atrial wall 104, by one or more actively engaging mechanisms or fixation mechanisms, such as a screw or helical member that screws into the myocardium. In an embodiment, the biostimulator 100 includes the fixation element 106 coupled to the housing 302. For example, the fixation element 106 can be mounted on a fixation element mount 307 that is in turn mounted on the housing 302.

The fixation element mount 307 can be mounted on a flange 308 of the housing 302, e.g., at a distal housing end 309. More particularly, the biostimulator 100 can include a header assembly having the flange 308 of the housing 302. The flange 308 can be coupled to the distal housing end 309 of a body of the housing 302. The fixation element 106 can be coupled to and extend distal to the flange 308. For example, the fixation element 106 and the fixation element mount 307 can be formed from a same material, e.g., titanium, and the fixation element 106 can be welded to the fixation element mount 307.

The fixation element 106 can extend about the longitudinal axis 304. For example, in the case of a helical fixation element, the fixation element 106 can include a helical wire 320 extending helically about the longitudinal axis 304. The helical wire 320 can spiral about the longitudinal axis 304 to a distal tip 322. Accordingly, when the biostimulator 100 is delivered to the target tissue, the distal tip 322 of the fixation element 106 can pierce the tissue and the housing 302 can be rotated or pushed to affix the fixation element 106 to the target tissue.

In an embodiment, the biostimulator 100 includes an attachment feature 310. The attachment feature 310 can be mounted on a proximal housing end 313 of the housing 302. More particularly, the attachment feature 310 can be mounted on an opposite end of the housing 302 from the fixation element 106, which as described above, can be coupled to the distal housing end 309 of the housing 302. The attachment feature 310 can facilitate precise delivery or retrieval of the biostimulator 100. For example, the attachment feature 310 can be formed from a rigid material to allow a transport system to engage the attachment feature 310 and transmit torque and/or axial loads to the attachment feature to plunge the fixation element 106, which may act as the pacing element, into the target tissue.

Referring to FIG. 4, a perspective view of a distal portion of a biostimulator having a tapering fixation element is shown in accordance with an embodiment. The fixation element 106 can extend distally to the distal tip 322, which can act as a pacing tip. More particularly, the helical wire 320 can revolve about the longitudinal axis 304 to the distal pacing tip. Accordingly, when the fixation element 106 screws into or otherwise engages the target tissue, the pacing element can also engage the tissue, and the housing 302 can be advanced and/or rotated to cause the distal pacing point of the pacing element to pierce the tissue and anchor the biostimulator 100.

The helical wire 320, which revolves about the longitudinal axis 304, can have several turns that include respective diameters. For example, the helical wire 320 can include a proximal turn 402, which is proximal to the distal tip 322. The helical wire 320 may include one or more intermediate turns between the proximal turn 402 and the distal tip 322. For example, the helical wire 320 can include a distal turn, distal to the proximal turn 402, that extends to the distal tip 322. The helical wire 320 may also have one or more intermediate turns longitudinally between the proximal turn 402 and the distal turn.

In an embodiment, the helical wire 320 tapers inward from the proximal turn 402 to the distal tip 322. For example, at any location along the helical wire 320, the location may have a respective radial distance to the longitudinal axis 304, and the radial distance may decrease in the distal direction. Accordingly, as described below, the helical wire 320 can spiral inward from the fixation element mount 307 toward the distal tip 322 such that the fixation element 106 at the distal tip 322 is nearer to the longitudinal axis 304 than at the proximal turn 402. The distal tip 322 may therefore remain close to the longitudinal axis 304 to pace tissue near the longitudinal axis 304 after implantation.

The fixation element 106 can be an active electrode, and can electrically communicate with the circuitry contained in the electronics compartment 306. For example, the fixation element 106 may electrically connect to the circuitry through an electrical feedthrough (not shown) disposed within the flange. Accordingly, the anchored element can electrically communicate with the tissue and can transmit electrical pulses between the tissue and the circuitry of the biostimulator 100.

The fixation element 106 can perform a dual function of fixation and pacing. More particularly, a portion of the fixation element 106 may be useful primarily for affixing and anchoring into the target tissue, and a portion of the fixation element 106 may be useful for transmitting electrical signals from or to electronic circuitry in the electronics compartment 306. The pacing function may benefit from a small conductive area, e.g., at the distal tip 322, and the anchoring function may benefit from insulating a proximal region of the fixation element 106 that grips the tissue. Accordingly, in an embodiment, at least a portion of the fixation element 106 of the biostimulator 100 is insulated and at least a portion of the fixation element 106 is exposed. For example, the fixation element 106 can have a proximal insulated section 404 and a distal exposed section 406. The proximal insulated section 404 can be longer than the distal exposed section 406 such that most of the helical wire 320 is masked behind the distal tip 322 or turn.

The exposed surface area of the distal tip 322 may be minimized or reduced to affect pacing capture thresholds. Furthermore, maintaining the distal tip 322 near the longitudinal axis 304 can reduce pacing capture thresholds. More particularly, the exposed surface area can correspond to an impedance of the electrode, and delivering pacing impulses near the longitudinal axis 304 without surrounding the distal tip 322 by a secondary helix can avoid transiently blocking a conductive path to the target tissue. Accordingly, the tapering fixation element 106 can both maintain good fixation and reduce pacing capture thresholds.

Proper impedance and/or pacing capture thresholds associated with the pacing element may be achieved by controlling an exposed length of the distal exposed section 406. In an embodiment, the distal exposed section 406 includes the distal tip 322 having an exposed length distal to the proximal insulated section 404. For example, the exposed length can be in a range of 0.2 mm to 2 mm, e.g., 1 mm. The exposed length may be measured along the longitudinal axis 304 and/or along a helical axis of the helical wire 320.

The proximal insulated section 404 can include an insulative outer layer surrounding a conductive core. For example, the proximal insulated section 404 may include an insulation sleeve surrounding the helical wire 320. The helical wire 320 can be formed from a stiff, conductive material, such as titanium, nickel titanium alloy, MP35N, or a combination thereof. The helical wire 320 can therefore conduct pacing impulses centrally through the proximal insulated section 404 to the distal tip 322 for pacing. The insulative sleeve 410 may include a flexible polymer tubing, e.g., a polytetrafluoroethylene tube or an ethylene tetrafluoroethylene tube mounted on the helical wire 320. Accordingly, the insulative sleeve 410 can surround, e.g., encapsulate, the helical wire conductor.

The insulation of the proximal insulated section 404 may alternatively include a coating on the helical wire 320. More particularly, the insulated section 404 can include an insulative coating 412 on the helical wire 320. The masking layer provided by the insulative coating 412 can be deposited on an adhesion layer. For example, the fixation element 106 can include a base layer of titanium nitride (TiNi) coating the helical wire 320 to alter a morphology and impedance of the helical wire surface. The TiNi layer can act as a tie layer that the insulation coating is deposited onto and held by. In an embodiment, the insulative coating 412 includes a parylene coating sprayed or otherwise deposited on the helical wire 320.

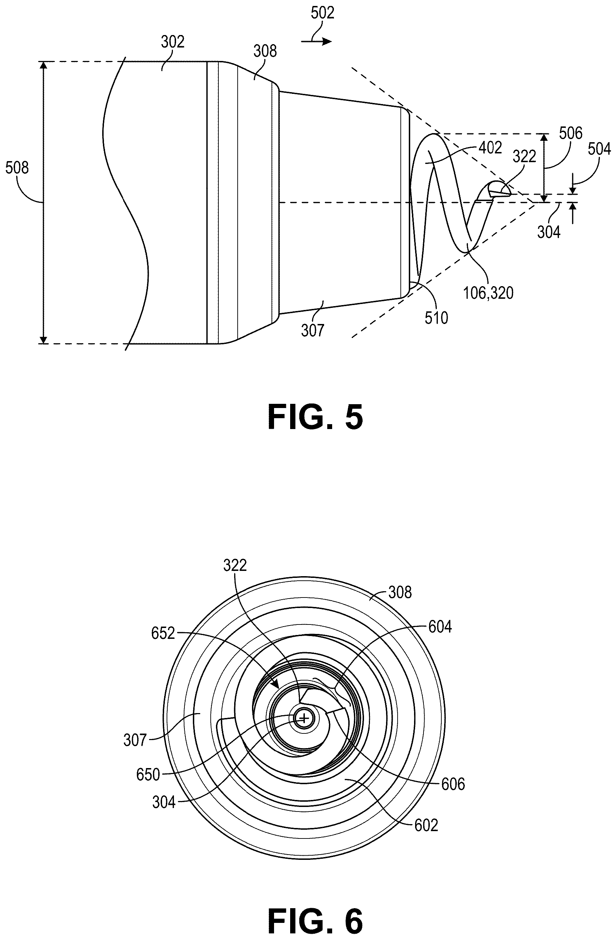

Referring to FIG. 5, a side view of a distal portion of a biostimulator having a tapering fixation element is shown in accordance with an embodiment. As described above, the fixation element 106 can taper toward the longitudinal axis 304 in a longitudinal direction 502. More particularly, the radial distance between the helical wire 320 and the longitudinal axis 304 may reduce over the helix length. For example, the helical wire 320 can have a first radial distance 504 from the distal tip 322 to the longitudinal axis 304, which may be less than a second radial distance 506 between the proximal turn 402 (or intermediate turn) of the helical wire 320 and the longitudinal axis 304. In an embodiment, the first radial distance 504 can be substantially less than the second radial distance 506, e.g., less than half of the second radial distance. The radial distance from the distal tip 322 to the longitudinal axis 304 may also have a predetermined relationship to a housing diameter 508 of the housing 302. For example, the first radial distance 504 can be less than one quarter of the housing diameter 508. The housing diameter 508 may be 6 cm and, thus, the first radial distance 504 may be 3 cm or less.

In any case, the radial distance of the helical wire 320 reduces distally over the length of the helical wire 320 to bring the distal tip 322 radially near to the longitudinal axis 304. For example, the first radial distance 504 can be 1 cm or less. Accordingly, the distal tip 322 can deliver pacing impulses to the target tissue along the longitudinal axis 304 and conductive pathways radially outward from the distal tip 322 may not be obstructed by secondary structures such as a surrounding fixation helix. Accordingly, the fixation element 106 can both anchor and pace the target tissue.

A degree of taper of the fixation element 106 can be influenced by an exposed length of the helical wire 320. In an embodiment, the exposed length, or height, can correspond to an expected thickness of a target anatomy. For example, the target anatomy can include the atrial wall 104, and the exposed length can be near an average thickness of the atrial wall 104. In an embodiment, the exposed length is a longitudinal distance between the distal tip 322 and a distal mount end 510 of the fixation element mount 307. The atrial thickness may be in a range of, for example, 2 mm to 2.5 mm. Accordingly, the distal tip 322 may be separated from the distal mount end 510 by a longitudinal distance in a range of 1.0 mm to 2.0 mm, e.g., 1.6 mm. The distal tip 322 can therefore engage the atrial wall 104 without passing fully through the atrial wall 104 in most cases. It will be appreciated, however, that the biostimulator 100 may be used in other, non-atrial, anatomies, which may be thicker than the atrial thickness. Accordingly, the separation distance may be greater than 2.0 mm, e.g., 3.0 mm, to accommodate thicker anatomical locations.

Referring to FIG. 6, an end view of a biostimulator having a tapering fixation element is shown in accordance with an embodiment. Optionally, the fixation element 106 can have a composite structure. More particularly, the helical wire 320 can be formed from a combination of two separate parts. In an embodiment, the helical wire 320 includes a proximal helical section 602 coupled to a distal helical section 604. For example, the proximal helical section 602 can be coupled to the distal helical section 604 at a joint. The proximal helical section 602 can include a first material, e.g., MP35N, and the distal helical section 604 can include a second material, e.g., platinum-iridium, and the dissimilar materials may be joined by the joint. For example, the joint can include a weld joint 606 and the helical sections may be coupled by the weld joint 606. Alternatively, an adhesive joint or a crimp joint may join the proximal helical section 602 to the distal helical section 604. In any case, the helical sections can form the helical wire 320 having sections of differing material characteristics.

The material characteristics of the helical sections may be suited to predetermined functions. For example, the proximal helical section 602 may be mechanically tougher than the distal helical section 604. By contrast, the distal helical section 604 may be less tough, but may have favorable electrical characteristics. For example, the distal helical section 604 can be formed from the platinum-iridium material to allow a TiNi coating to be deposited on the tip for favorable impedance and pacing characteristics.

In an embodiment, the distal helical section 604 is an exposed section, such as the distal exposed section 406 described above. By contrast, the proximal helical section 602 may be an insulated section, such as the insulated section 404 described above. Accordingly, the insulated section 404 may be joined to the exposed section 406 at a joint, e.g., the weld joint 606, and conductive cores of both sections may be electrically connected through the joint to conduct pacing impulses to the target tissue.

The biostimulator 100 can incorporate additional features to aid in anchoring and/or treatment. For example, one or more side facing sutures (not shown) may extend radially outward from an outer surface of the housing 302 to resist anti-rotation in a direction opposite to the rotation used during implantation. The side facing sutures can assist in securing the fixation element 106 in the target tissue.

In an embodiment, the biostimulator 100 include a monolithic controlled release device (MCRD) 650. The MCRD 650 can be a filler including a therapeutic agent contained within a carrier matrix. For example, the carrier matrix can be a silicone matrix, e.g., a monolithic silicone plug, which is impregnated with the therapeutic agent. The therapeutic agent can in turn be contained within pores of the silicone matrix. In at least one implementation, the therapeutic agent can include a corticosteroid, such as dexamethasone sodium phosphate, dexamethasone acetate, etc.

The fixation element 106 can extend helically about an interior 652. For example, the interior 652 can be a space radially inward from the fixation element 106 along the longitudinal axis 304. The MCRD 650 may be within the interior 652, e.g., positioned along the longitudinal axis 304. The MCRD 650 may include, for example, a plug inside a recess formed within the fixation element mount 307. The plug may elute the therapeutic agent when blood or tissue enters the interior 652. Accordingly, the MCRD 650 can elute therapeutic agent through the interior 652 and into the target tissue engaged by the distal tip 322.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a housing (302) having a longitudinal axis (304) and containing an electronics compartment (306);
a fixation element mount (307) mounted on the housing (302); and
a fixation element (106) mounted on the fixation element mount (307), wherein the fixation element (106) includes a helical wire (320) extending helically about the longitudinal axis (304), and wherein the helical wire (304) tapers inward from a proximal turn (402) to a distal tip (322).

2. The biostimulator of claim 1, wherein a radial distance (504) from the distal tip (322) to the longitudinal axis (304) is less than one quarter of a housing diameter (508) of the housing (302).

3. The biostimulator of claim 2, wherein the radial distance (504) is 1 cm or less.

4. The biostimulator of any one of claims 1 to 3, wherein the fixation element mount (307) has a distal mount end (510), and wherein the distal tip (322) is separated from the distal mount end (510) by a longitudinal distance in a range of 1.0 mm to 3.0 mm.

5. The biostimulator of any one of claims 1 to 4, wherein the fixation element (106) has a proximal insulated section (404) and a distal exposed section (406), and wherein the distal exposed section (406) includes the distal tip (322).

6. The biostimulator of claim 5, wherein the distal exposed section (406) has an exposed length in a range of 0.2 mm to 2 mm.

7. The biostimulator of claim 5 or 6, wherein the proximal insulated section (404) includes an insulative sleeve (410) surrounding the helical wire (320).

8. The biostimulator of claim 7, wherein the insulative sleeve (410) includes a flexible polymer tubing mounted on the helical wire (320).

9. The biostimulator of claim 5 or 6, wherein the proximal insulated section (404) includes an insulative coating (412) on the helical wire (320).

10. The biostimulator of any one of claims 1 to 9, wherein the helical wire (320) includes a proximal helical section (602) coupled to a distal helical section (604).

11. The biostimulator of claim 10, wherein the proximal helical section (602) includes a first material and wherein the distal helical section (604) includes a second material different than the first material.

12. The biostimulator of claim 10 or 11, wherein the proximal helical section (602) is coupled to the distal helical section (604) by a weld joint (606).

13. The biostimulator of any one of claims 1 to 12, wherein the fixation element (106) extends helically about an interior (652).

14. The biostimulator of claim 13 further comprising a monolithic controlled release device (650) within the interior (652).

15. A biostimulator system (200), comprising:
a biostimulator transport system (202); and
the biostimulator (100) of any one of claims 1 to 14 mounted on the biostimulator transport system (202).
